# EUROPEAN PATENT SPECIFICATION

(11) **EP 2 978 164 B1**
(45) Date of publication and mention of the grant of the patent: **29.05.2019**
(21) Application number: 14178640.0
(22) Date of filing: 25.07.2014
(51) Int. Cl.: G16H 40/20

(54) **Hybrid nurse call system**
Hybrides Pflegerufsystem
Système d'appel d'infirmière hybride

(43) Date of publication of application: 27.01.2016
(73) Proprietor: Televic Healthcare NV, 8870 Izegem (BE)
(72) Inventor: Crombez, Pieter, B-8820 Torhout (BE); Gesquiere, John, B-8970 Poperinge (BE); Verhaeghe, Geert, B-8680 Koekelare (BE); Desmet, Ludwig, B-8770 Ingelmunster (BE)
(74) Representative: Beck, Michaël Andries T.

(56) References cited:
- WO-A2-2005/057834
- US-A1- 2008 097 908
- US-A1- 2009 212 925

## Description

### Field of the Invention

The present invention relates to the field of communication systems, more in particular to the field of nurse call systems as used in hospitals and similar facilities.

### Background

Nurse call systems are communication networks designed to relay, *inter alia,* patient localization information and distress calls to a central system, which in turn pages personnel to respond to the situation or the call.

As nurse call systems should allow people to quickly call assistance in emergency situations, security against system failure and the ability to operate flawlessly at all times are of utmost importance. The VDE standard (DIN VDE 0834) takes these considerations into account. It prescribes that the call system be completely independent of elements that are not part of the system. Data transfer with other systems is only allowed when using the system's own interfaces.

Accordingly, for safety reasons, nurse call systems are required to be able to fall back from their normal "networked operation" mode to a "local operation" mode in the event of a loss of network connectivity. In such a case, the hardware will provide highly noticeable visual clues indicating that "local operation" mode is active, and, if necessary, use audio-visual signaling to indicate the presence of a call or the need for a personnel intervention.

It is a disadvantage of the known nurse-call systems that one or more localized network defects can seriously impair the system's ability to continue working in "networked operation" mode.

It is therefore an object of embodiments of the present invention to at least partially overcome said disadvantage.

### Summary of the Invention

According to an aspect of the present invention, there is provided a method for distributing information in a nurse call localization system, the method comprising at a first beacon apparatus adapted to receive identification messages originating from an identification tag via a first radio frequency transceiver and connected to a wired backbone network: detecting a need to relay the information to a server; testing the wired backbone network for the presence of certain critical defects; if none of the critical defects are found to be present, transmitting the information to the server over the wired backbone network; and otherwise, using the radio frequency transceiver to transmit the information to the server.

It is an advantage of the present invention that the nurse call system does not have to fall back to a completely local operation mode when a defect occurs on the wired backbone network. The beacon apparatus according to the present invention retains the ability to transmit a message to the server, when it detects a need to do so. In the case of a critical defect of the wired backbone network, the system advantageously reuses the radio frequency transceiver of the beacon apparatus, normally used to receive identification messages from patient or equipment tags, to establish outgoing wireless communication with the server.

In an embodiment of the method according to the present invention, the detecting of the need comprises receiving a message comprising information to be relayed to the server.

The beacon apparatus may be requested to pass on information to the server, by a device that has no connection with the server itself. It is an advantage of this embodiment that the beacon apparatus' transmission stage is triggered by the receipt of such a request to relay information.

In a particular embodiment, the message is an identification message originating from the identification tag.

Relaying information pertaining to the location and identity of identification tags residing within range (e.g. ultrasound range or RF range) of the beacon apparatus, may be one of the core functions of the beacon apparatus. It is an advantage of this embodiment that the beacon apparatus is used to relay such information to the server via the wireless medium, even if the wired backbone network is impaired.

In a particular embodiment, the message is a message transmitted by a similar beacon apparatus.

It is an advantage of this embodiment that another beacon apparatus that detects a critical defect of the wired backbone network, and that is outside the RF range of the server, may request another beacon apparatus to act as a next step in a multi-beacon relay that will eventually reach the server.

In an embodiment of the method according to the present invention, the detecting of the need comprises detecting a presence of one or more of the critical defects.

It is an advantage of this embodiment that the very detection of a critical defect on the wired backbone network will trigger an alarm to be transmitted to the server. As, in this hypothesis, the wired backbone network is not available to relay the alarm, the alarm will be transmitted by means of the radio-frequency transceiver.

In an embodiment of the method according to the present invention, the detecting of the need comprises receiving a command from a user via a user interface comprised in the beacon apparatus.

It is an advantage of this embodiment that the beacon apparatus can be made to send a message to the server at the request of a user, for instance upon pushing a button, scanning a badge, reading a finger print, and the like.

In an embodiment of the method according to the present invention, the detecting of the need comprises detecting an expiry of a timer.

It is an advantage of this embodiment that the beacon apparatus can be made to transmit a "keep-alive" message at regular intervals. The server, or other appropriate network elements, may be configured to respond to these "keep-alive" messages by means of acknowledgment messages. These acknowledgment messages are indicative of proper operation of all intervening equipment. If an acknowledgment of a "keep-alive" message (or a pre-determined number of "keep-alive" messages) is missed, this may be indicative of a defect of the wired backbone network, and this may constitute the detection of a critical defect as referred to above.

In an embodiment of the method according to the present invention, the server comprises a second radio frequency transceiver, and the use of the radio frequency transceiver comprises transmitting the information to the second radio frequency transceiver.

It is an advantage of this embodiment that point-to-point wireless communication can take place directly between the beacon apparatus and the server, without the need for intervening network nodes.

In an embodiment of the method according to the present invention, the use of the radio frequency transceiver comprises relaying the information to a similar beacon that has unimpeded access to the wired backbone network.

It is an advantage of this embodiment that the beacon apparatus can reach the server, even when the latter is not within radio-transmission range of the former, by using another beacon apparatus as a relay. The other beacon apparatus may have unimpaired connectivity to the wired backbone network, or it may be better positioned to establish wireless communication with the server (if the server has a wireless interface) or with a dedicated wireless gateway.

In an embodiment of the method according to the present invention, the use of the radio frequency transceiver comprises relaying the information via a series of similar beacons to a similar beacon that has unimpeded access to the wired backbone network.

It is an advantage of this embodiment that the beacon apparatus can reach the server, even when the latter is not within radio-transmission range of the former, by using a better positioned beacon apparatus as a relay. The other beacon may pass on the message via the wireless medium to yet another beacon apparatus that has unimpaired connectivity to the wired backbone network, or that is better positioned to establish wireless communication with the server (if the server has a wireless interface) or with a dedicated wireless gateway. The relaying process may be repeated as often as necessary; thus, a series of beacon apparatus can pass the communication on to the next network node that has direct or indirect connectivity with the server.

The method according to the present invention further comprises transmitting at regular intervals a beacon message from the first beacon apparatus via one of the radio frequency transceiver, a magnetic induction transceiver, and an ultrasound transmitter; and, upon detection of one or more of the certain critical defects of the wired backbone network at the first beacon or at a similar beacon with wireless communication range of the first beacon, increasing the duration of the intervals.

Advantageously, the inventive method can be applied to an operational beacon apparatus of a patient localization system or equipment localization system, while minimizing the impact of the re-use of the radio-frequency transceiver on the normal operation of the localization system. This is achieved by judiciously sharing the available airtime between the localization protocol and the beacon-to-server communication. In particular, this is achieved by reducing the beacon message and identification message frequency whenever the need to communicate with the server via the wireless medium is established.

In an embodiment, the method according to the present invention further comprises, if one or more of the critical defects is found to be present, emitting a signal.

It is an advantage of this embodiment that personnel or the server will immediately be made aware of the presence of the critical defect, such that an appropriate intervention can be launched. If intended for personnel, the signal may be visual, auditory, or a combination thereof. If intended for the server, the signal may be a suitably formatted message including information about the location and the nature of the critical defect.

In an embodiment, the method according to the present invention further comprises using the radio frequency transceiver to receive additional information from the server, the additional information having been relayed by a third beacon apparatus.

It is an advantage of this embodiment that the communication between the beacon apparatus and the server can be made bidirectional. Information from the server may reach the beacon apparatus via the reverse of the path that was taken by the message that was sent from the beacon apparatus to the server. In particular, it may involve relaying of the information by one or more beacon apparatus. The reverse path may be set up proactively when the message is sent to the server, either by storing forwarding information at the intermediate nodes (leaving virtual breadcrumbs), or by adding path information to the message as it is being relayed, such that the server receives complete information about an available path for reverse communication along with the message. The server may store each such path in a route cache for a predetermined limited time.

According to an aspect of the present invention, there is provided a computer program product comprising code means configured to cause a processor to carry out the method as described above. According to an aspect of the present invention, there is provided a beacon apparatus for use in a nurse call localization system, the beacon apparatus comprising a first radio frequency transceiver adapted to receive identification messages originating from an identification tag, and a network interface connectable to a wired backbone network; the beacon apparatus being configured to transmit information to a server over the wired backbone network; wherein the beacon apparatus further comprises a diagnostic agent configured to detect certain critical defects of the wired backbone network; and wherein the beacon apparatus is further configured to use the first radio frequency transceiver to transmit the information to the server if the diagnostic agent detects the presence of one of the critical defects of the wired backbone network; and wherein said beacon apparatus is configured to transmit at regular intervals a beacon message via one of said first radio transceiver, a magnetic induction transceiver, and an ultrasound transmitter; and to increase the duration of said intervals if said diagnostic agent detects the presence of one of said critical defects of said wired backbone network.

The technical effects and advantages of embodiments of the beacon apparatus and the computer program product according to the present invention correspond *mutatis mutandis* to those of the corresponding embodiments of the method according to the invention.

WO 2005/057834 A2 discloses a plug-in network appliance for a wireless networking system, having a transceiver to receive and transmit data on wired and wireless networks according to possibly more than one communication format (bridging function),while receiving power from an outlet. Applications include also networks in hospitals and tracking systems.

US 7,153,263 B2, assigned to GE Medical Systems Information Technologies, Inc., discloses a wireless local area network (WLAN) system comprising multiple access points that are distributed throughout a medical facility to provide wireless access to a hardwired network. The access points implement multiple WLAN protocols, including a real-time protocol for real-time patient monitoring (telemetry) and a standard WLAN protocol (such as IEEE 802.11 within an ISM band) for providing general-purpose wireless access. Some or all of the access points preferably implement both WLAN protocols such that the different WLANs and wireless device types share network access resources. Some or all of the access points may also include RF location-tracking modules which may be used to track locations of patients, hospital personnel, capital equipment, and/or disposable medical supplies. Also disclosed are an antenna design which may be used with the access points to improve reception (particularly for patient monitoring), and a TDMA timeslot rotation method for avoiding lockstep interference between access points that operate on the same channel. That document does not disclose detecting critical defects on a wired backbone network or using an RF interface, normally intended for localization purposes, for communication with a server.

### Brief Description of the Figures

These and other features and advantages of embodiments of the present invention will now be described in more detail with reference to the accompanying drawings, in which:
Figure 1 schematically illustrates a network in which embodiments of the present invention may be used;
Figure 2 schematically illustrates a network in which embodiments of the present invention may be used, including additional details;
Figure 3 illustrates a typical configuration in a building, such as a hospital building, in which embodiments of the present invention may be used;
Figure 4 illustrates an embodiment of the invention, in which a beacon apparatus communicates with a server via a wireless link with another beacon apparatus;
Figure 5 illustrates an embodiment of the invention, in which a beacon apparatus communicates with a server via a wireless link with a series of other beacon apparatus;
Figure 6 illustrates a typical room configuration, such as a hospital room, in which embodiments of the present invention may be used;
Figure 7 provides a flow chart of a method according to an embodiment of the present invention; and
Figure 8 provides a flow chart indicating additional steps of a method according to an embodiment of the present invention.

### Detailed Description of Embodiments

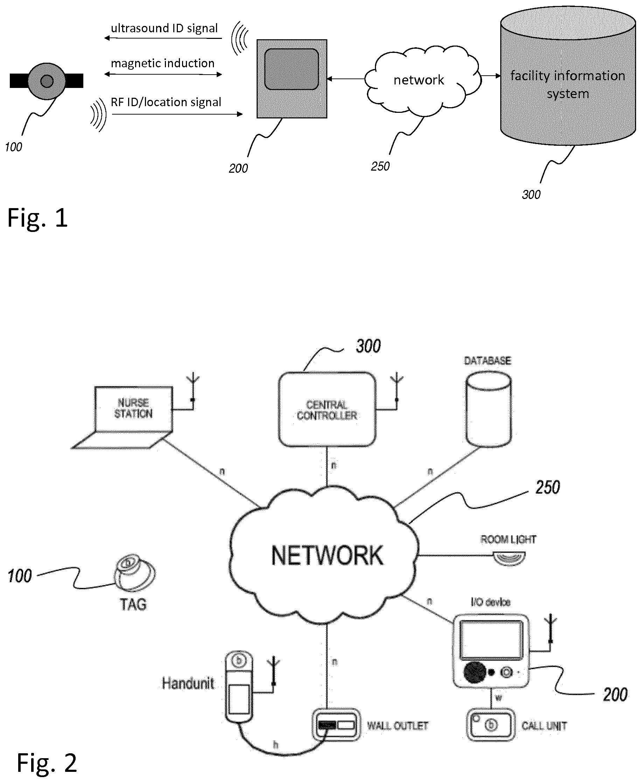

Figure 1 schematically illustrates a nurse call localization system in which embodiments of the present invention may be used.

A specific application of the location system described herein is a wireless nurse call system for use in hospitals and other institutions where patients may move about, possibly without being fully conscious of their own exact location. Where appropriate, the invention will be described with reference to such a nurse call system, without intent to limit the scope of the invention to such applications. In the context of a wireless nurse call system, the development of efficient hardware and efficient communication protocols is an important goal, with a view to reducing (battery) power consumption, obtaining a small form factor, and keeping the total cost as low as possible.

In the preferred localisation system, beacons **200** are provided at fixed locations throughout an area in which the location of mobile objects or persons is to be monitored. The beacons may generally be mounted to walls, doors, pillars, and the like. They may have a basic user interface comprising a display and one or more keys.

The beacons emit an identification element, which is preferably modulated onto an ultrasound signal. Ultrasound communication is based on electromechanically induced vibrations that generate propagating longitudinal acoustic waves. As ultrasound waves are, by definition, in a frequency range beyond the audible range for humans, their use is no hindrance to humans present in the monitored area.

Within building environments, ultrasound waves are almost completely blocked by walls (contrary to electromagnetic waves). Ultrasound waves are therefore a suitable signal type to obtain room-level localisation accuracy. With additional processing, even sub-room-level accuracy may be achieved.

However, it is also possible to transmit the beacon signals in other forms, such as low-power RF signals. The power of the RF signals is preferably calibrated in such a way that the probability of any tag receiving such a signal in an adjacent room is virtually zero. In yet another alternative, the beacon signals may be transmitted by means of magnetic induction communication.

The mobile objects or persons to be monitored are provided with identification tags (hereinafter also referred to as "tags") **100**, which comprise a receiver for the signals emitted by the beacons **200**, *i*.*e*., in the case of ultrasound transmission, an ultrasound receiver.

Upon receiving the beacon identification element encoded in the (ultrasound) signal, the tag **100** will be aware of its location (in the sense of being able to identify the nearest beacon **200)** down to room-level accuracy, without any need for triangulation.

The tag **100** further comprises communication means to relay the decoded beacon identification element, along with its own identity, to the central monitoring system, in the form of a localisation message. The communication means may include a radio frequency (RF) transmitter adapted to wirelessly communicate the information to a beacon (the same beacon whose identification element was received and/or another beacon within radio range), which is in turn connected to a wired network **250** that allows it to communicate with a centralized management system or server **300**.

The beacon apparatus **200** is adapted to receive the identification messages from the tag **100** via a radio frequency transceiver. The nurse call system as considered in this invention can be expanded to cater different needs such as intercom, domotics control, access control, asset tracking and more, and the beacon apparatus **200** will be equipped accordingly.

This first beacon apparatus **200** is connected to a wired backbone network **250**, which connects the beacon apparatus **200** to a server **300.** The wired backbone network **250** is typically a Local Area Network (LAN), which may comprise segments that comply with various LAN standards. At the physical and data link layer, the network advantageously comprises a bridged LAN including segments that operate according to IEEE Std 802.3 (commonly referred to as "Ethernet", which includes specifications for communication over twisted pair cabling, coaxial cabling, and optical fiber). At the network layer, communication may take place by means of the Internet Protocol (IP). The skilled person will appreciate that various other standardized and proprietary networking protocols may be used instead. The use of standardized network equipment simplifies service and allows easy exchange or expansion of devices in rooms. Additional nurse call functions such as VoIP-telephony can easily be implemented.

While traditional Ethernet-based networks are known to be nondeterministic, they can be used for mission critical applications. Ethernet can be used if sufficient bandwidth is provided in view of expected peak loads, and if adequate backup communication means are present. However, industry practices require that all communication infrastructure is provided by the vendor of the nurse call system, in order to keep the responsibility for the correct functioning of the system unambiguous. Thus, if third-party network equipment is used, it is highly desirable to also have a back-up path made up entirely of equipment provided by the vendor of the nurse call system. The present invention is based *inter alia* on the insight of the inventors that a nurse call system may be designed in such a way that the radio-frequency transceivers that are already present in the beacon apparatus may assume the role of back-up communication means.

From time to time, the beacon apparatus **200** is engaged to transmit information to the server **300**. Such transmissions are normally effected via the wired backbone network **250**. Embodiments of the present invention take possible impairments of the wired backbone network **250** into account in their transmission algorithm. To this end, the beacon apparatus **200** tests the wired backbone network **250** for the presence of certain critical defects. These critical defects may include a loss of connection at the level of the physical or data link layer, network congestion (detected for example by the receipt of flow control messages from the other end of the link), or the absence of expected acknowledgment messages after certain transmissions. If none of the critical defects are found to be present, the information is transmitted to the server **300** over the wired backbone network **250.** Otherwise, the radio frequency transceiver, which is normally intended for receiving identification messages originating from identification tags **100**, will be used to transmit the information to the server **300.**

Figure 2 schematically illustrates a nurse call system with additional components. A hand call and/or handset may be connected to a wall outlet via a flexible network cable **h.** Other network connections (not necessarily flexible) are designated in the drawing by the symbol **n.** The outlet connects the handset cable to the network. In the topology of Figure 2, the call unit is in connection with the I/O device via a passive link **w.** The I/O device itself is connected to the network through a network cable **n.** An I/O device may have display capabilities (e.g., an LCD or a touchscreen) and act as a terminal.

Other components of the nurse call system that are shown in Figure 2 include a nurse station, a central controller and a database. These components are also linked with each other through network connections **n.** The database allows storage of information concerning any errors detected in the nurse call system, which can be useful for later analysis. Immediately required interventions are typically displayed at the nurse station. Additionally or alternatively, these messages may be forwarded by the central controller to third-party systems like digital cellular phones, DECT handsets, pagers, and the like.

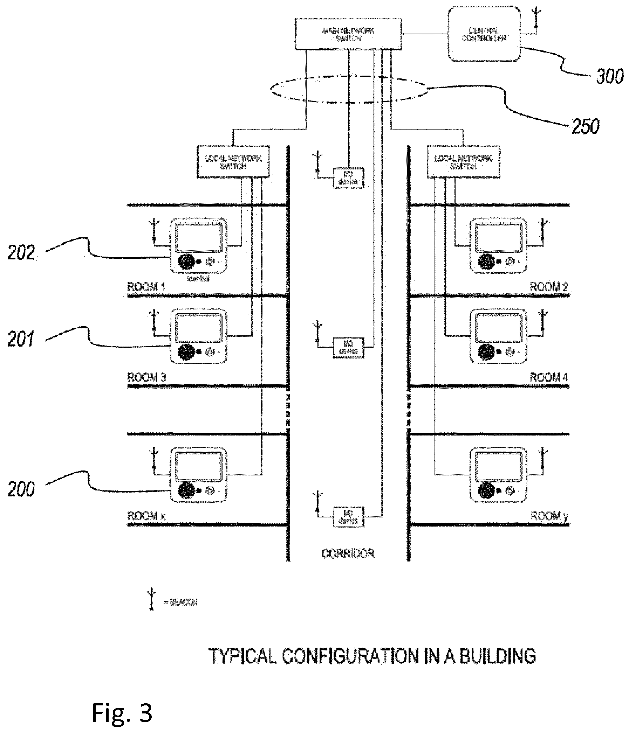

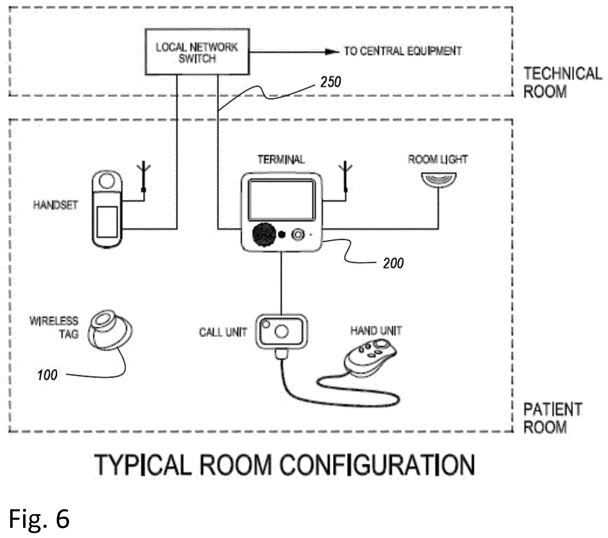

A vital part in a nurse call system is formed by the devices the patients and nursing staff have access to, hereafter called user devices. The principal user devices are considered to be the I/O device **200,** the hand unit, the central controller **300** and the nurse station. As can be seen in Figure 6 users devices are typically found in a patient room. This room is usually part of a larger building as is illustrated in Figure 3. Depending on the needs and wishes of the users, extra I/O devices may be located in this building outside the patient rooms. The number and layout of the rooms and their beacon apparatus as illustrated in the Figures is purely exemplary, and not intended to limit the scope of the invention.

The user devices (and, optionally, the central controller) include beacons, which can send and receive wireless messages. Devices including a beacon are referred to herein as "beacon apparatus" **200, 201, 202.**

When a user presses a call button **B** located on a tag **100,** a hand unit or an I/O device **200,** a nurse call message (also referred to as an alarm) is transmitted to the central controller **300** through the network **250** for processing and distributing. Depending on the outcome of this processing, one or more of the aforementioned buttons may be lit by passing network messages from the central controller **300** to the appropriate user device(s). Optionally, the alarm messages are visualized on I/O devices having other display possibilities like an LCD or room lamp **L.**

If a user presses a call button on a user device (*e*.*g*., a call unit), the call is transferred to the I/O device **200** (a beacon apparatus) through the passive connection **w,** in a manner similar to the normal operation described above. Upon receipt of the alarm at the I/O device **200,** this device will check if its network connection **250** to the central controller **300** is still in service. If this is not the case, the I/O device **200** passes the alarm to the incorporated beacon, the RF transceiver of which will be used to transmit the message.

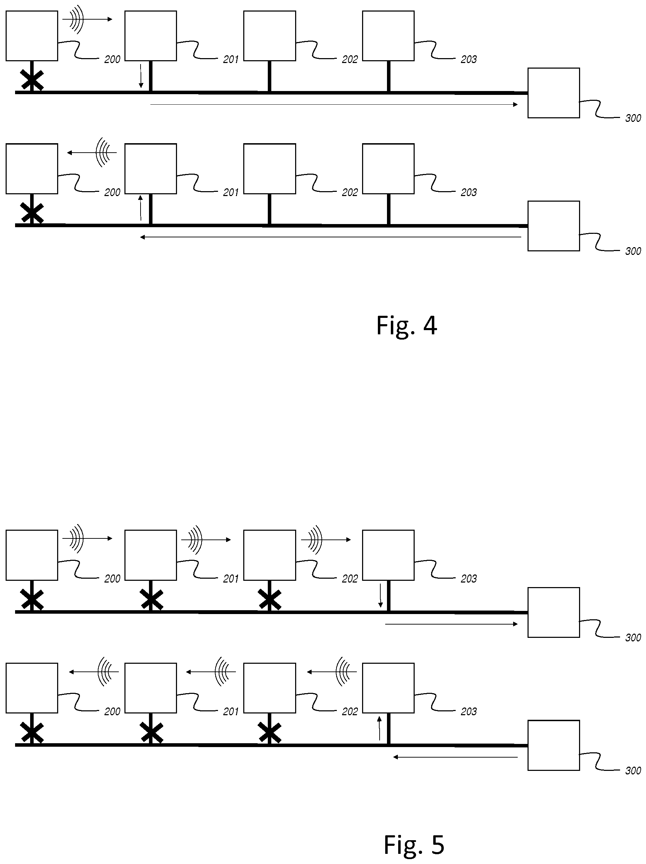

In a first scenario, illustrated in Figure 4 (top), the RF transceiver issues a wireless transmission incorporating the alarm message to another beacon **201**, which has an unimpaired connection to the wired backbone network **250**.

In a second scenario, illustrated in Figure 5 (top), the RF transceiver issues a wireless transmission incorporating the alarm message to another beacon **201**, which has the capability to relay the message further towards the central controller **300** via its radio-frequency interface, by relaying the message to other beacon apparatus **201, 202, 203** until a valid wired network connection is encountered and the message can be transmitted over the wired network **250** to the controller **300**. The illustrated number of intermediate beacon apparatus is purely exemplary and presented without loss of generality.

In case the central controller **300** is equipped with its own beacon, the central controller **300** may be reached by the last beacon apparatus in the chain (beacon apparatus **202** in Figure 4 or beacon apparatus **203** in Figure 5) via wireless transmission.

If the server **300** cannot be reached in the manner described above, this is preferably indicated to the users and/or personnel, independently of the presence of an alarm call, by a special audiovisual indications (e.g., using the room light).

A similar fallback mechanism can be activated if the controller **300** needs to send a message to an I/O device **200** with an impaired network connection, as illustrated in Figure 4 (bottom) and Figure 5 (bottom). The controller **300** will send the message to an appropriate reachable I/O device (e.g., beacon **201** in Figure 4 or beacon **203** in Figure 5) that is selected on the basis of its (topological or physical) proximity to the destination device **200.**

The controller will base its decision on static network information, such as physical network topology (the locations, interconnections, and/or RF ranges of devices), and/or dynamic network information, such as detected network errors and routes used by incoming messages. When the intermediate I/O device **202** receives the message from the controller **300,** it will detect that is not for its own usage, but to be relayed. One or more further intermediate beacon devices **201** may be involved in relaying the message to the destination device **200** using the same mechanism as described above for the originating call transmission.

When the beacon apparatus **200** is in a situation where it relays messages via its radio frequency interface, as described above, it may apply queuing rules that assign different priorities to different types of traffic. Hence, doctor call messages intended for the server **300** may be given a higher priority than nurse call messages, which in turn may be given a higher priority than regular location updates.

To facilitate the relaying of messages through one or more beacon apparatus, it is useful if individual beacons can find out the topology of the wireless network. Accordingly, the method according to the invention may further comprise transmitting a discovery message via the radio frequency transceiver, and receiving a response to the discovery message from similar beacons that are within wireless communication range. A target for the relaying is selected from among the beacons that are within wireless communication range. The beacons that receive the discovery message, transmit a response that includes information pertaining to the detection of the certain critical defects of the wired backbone network at their location, and/or information pertaining to similar beacons within wireless communication range (which they may have received through their own discovery exchanges).

The information gathered by means of these discovery messages and responses may be combined at the server with pre-existing information about the lay-out of the facility, to produce a global and up-to-date topological map of the ad-hoc network created by the radio frequency interfaces of the beacon apparatus. This map allows the server to determine optimal routes to address messages to individual beacons apparatus.

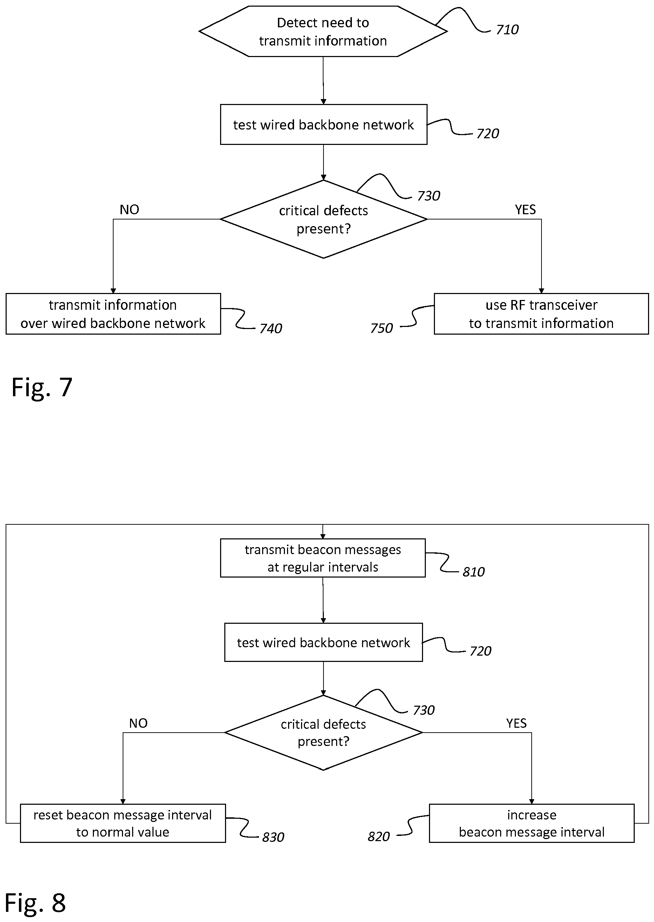

Figure 7 provides a flow chart of a method according to an embodiment of the present invention. Where relevant, reference will be made to the figure references introduced in the description of the previous figures.

The flow chart represents a method for distributing information in a nurse call localization system. The following steps are carried out at a beacon apparatus **200** adapted to receive identification messages originating from an identification tag **100** via a radio frequency transceiver and connected to a wired backbone network **250.** The illustrated method starts when a need to relay information to the server **300** is detected **710.** The wired backbone network **250** is tested **720** for the presence of certain predetermined critical defects; this may happen prior to each attempted transmission, or at regular intervals, regardless of whether there is data to be transmitted at that time. If **730** none of said critical defects are found to be present, the information is transmitted **740** to the server **300** over the wired backbone network **250.** Otherwise, the radio frequency transceiver of the beacon apparatus **200** is used **750** to transmit the information to the server **300.**

Figure 8 provides a flow chart indicating additional steps of a method according to an embodiment of the present invention, which is in particular used as a patient or equipment localization system.

This embodiment further comprises transmitting **810** at regular intervals a beacon message from said first beacon apparatus **200** via one of said radio frequency transceiver, a magnetic induction transceiver, and an ultrasound transmitter. Upon detection **730** of one or more of said certain critical defects of the wired backbone network **250,** at said first beacon **200** or at a similar beacon with wireless communication range of said first beacon **200,** the duration of these intervals is increased **820.** This frees up the necessary airtime for the radio-frequency transceivers to act as a backup communication means for the backbone network **250.**

While the invention has been described hereinabove with reference to specific embodiments, this is done to illustrate and not to limit the invention, the scope of which is defined by the accompanying claims.

## Claims

1. A method for distributing information in a nurse call localization system, the method comprising at a first beacon apparatus (200) adapted to receive identification messages originating from an identification tag (100) via a first radio frequency transceiver and connected to a wired backbone network (250) :
- detecting (710) a need to relay said information to a server (300) ; **characterized by**:
- testing (720) said wired backbone network (250) for the presence of certain critical defects;
- if (730) none of said critical defects are found to be present, transmitting (740) said information to said server (300) over said wired backbone network (250); and
- otherwise, using (750) said radio frequency transceiver to transmit said information to said server (300);
wherein the method further comprises:
- transmitting (810) at regular intervals a beacon message from said first beacon apparatus (200) via one of said radio frequency transceiver, a magnetic induction transceiver, and an ultrasound transmitter; and, upon detection (730) of one or more of said certain critical defects of said wired backbone network (250) at said first beacon (200) or at a similar beacon with wireless communication range of said first beacon (200), increasing (820) the duration of said intervals.

2. The method according to claim 1, wherein said detecting (710) of said need comprises receiving a message comprising information to be relayed to said server (300).

3. The method according to claim 2, wherein said message is an identification message originating from said identification tag (100) .

4. The method according to claim 2, wherein said message is a message transmitted by a similar beacon apparatus (201).

5. The method according to claim 1, wherein said detecting (710) of said need comprises detecting a presence of one or more of said critical defects.

6. The method according to claim 1, wherein said detecting (710) of said need comprises receiving a command from a user via a user interface comprised in said beacon apparatus (200).

7. The method according to claim 1, wherein said detecting (710) of said need comprises detecting an expiry of a timer.

8. The method according to any of claims 1-7, wherein said server (300) comprises a second radio frequency transceiver, and wherein said use (750) of said radio frequency transceiver comprises transmitting said information to said second radio frequency transceiver.

9. The method according to any of claims 1-7, wherein said use (550) of said radio frequency transceiver comprises relaying said information to a similar beacon (201) that has unimpeded access to said wired backbone network (250).

10. The method according to any of claims 1-7, wherein said use (750) of said radio frequency transceiver comprises relaying said information via a series of similar beacons (201, 202) to a similar beacon that has unimpeded access to said wired backbone network (250).

11. The method according to any of the preceding claims, further comprising, if one or more of said critical defects is found to be present, activating a visual signal.

12. The method according to any of the preceding claims, further comprising using said radio frequency transceiver to receive additional information from said server (300), said additional information having been relayed by a third beacon apparatus (202, 203) .

13. A computer program product comprising code means configured to cause a processor to carry out the method according to any of the preceding claims.

14. A beacon apparatus (200) for use in a nurse call localization system, the beacon apparatus (200) comprising a first radio frequency transceiver adapted to receive identification messages originating from an identification tag (100), and a network interface connectable to a wired backbone network (250); the beacon apparatus (200) being configured to detect a need to relay said information to a server ; **characterized in that**:
said beacon apparatus (200) further comprises a diagnostic agent configured to detect certain critical defects of said wired backbone network, said beacon apparatus being configured to transmit said information over said wired backbone network if none of said critical defects are found to be present;
said beacon apparatus (200) is further configured to use said first radio frequency transceiver to transmit said information to said server (300) if said diagnostic agent detects the presence of one of said critical defects of said wired backbone network (250); and
said beacon apparatus (200) is further configured to transmit at regular intervals a beacon message via one of said first radio frequency transceiver, a magnetic induction transceiver, and an ultrasound transmitter; and to increase the duration of said intervals if said diagnostic agent detects the presence of one of said critical defects of said wired backbone network (250).

## Patentansprüche

1. Verfahren zum Verbreiten von Informationen in einem Lokalisierungssystem für Rufe von Pflegepersonal, wobei das Verfahren an einer ersten Bakenvorrichtung (200), die für das Empfangen von Identifikationsmeldungen ausgelegt ist, die von einem Identifikationstag (100) ausgehen, über einen ersten Funkfrequenzsendeempfänger und mit einem verdrahteten Backbone-Netzwerk (250) verbunden, umfasst:
- Erkennen (710) einer Notwendigkeit die Informationen an einen Server (300) weiterzuleiten, **gekennzeichnet durch**:
- Testen (720) des verdrahteten Backbone-Netzwerks (250) auf Vorliegen bestimmter kritischer Defekte;
- falls (730) festgestellt wird, dass keine der kritischen Defekte vorliegen, Senden (740) der Informationen an den Server (300) über das verkabelte Backbone-Netzwerk (250); und
- andernfalls, Verwenden (750) des Funkfrequenzsendeempfängers zum Senden der Informationen an den Server (300);
wobei das Verfahren ferner umfasst:
- Senden (810), in regelmäßigen Intervallen, einer Bakenmeldung von der ersten Bakenvorrichtung (200) über eines von dem Funkfrequenzsendeempfänger, einem Magnetinduktionssendeempfänger und einem Ultraschallsender; und, bei Erkennung (730) von einem oder mehreren der bestimmten kritischen Defekte des verdrahteten Backbone-Netzwerks (250) an der ersten Bake (200) oder an einer ähnlichen Bake mit drahtlosem Kommunikationsbereich der ersten Bake (200), Erhöhen (820) der Dauer der Intervalle.

2. Verfahren nach Anspruch 1, wobei das Erkennen (710) der Notwendigkeit Empfangen einer Meldung umfasst, die an den Server (300) weiterzuleitende Informationen umfasst.

3. Verfahren nach Anspruch 2, wobei die Meldung eine Identifikationsmeldung ist, die von dem Identifikationstag (100) ausgeht.

4. Verfahren nach Anspruch 2, wobei die Meldung eine Meldung ist, die von einer ähnlichen Bakenvorrichtung (201) gesendet wird.

5. Verfahren nach Anspruch 1, wobei das Erkennen (710) der Notwendigkeit Erkennen eines Vorliegens eines oder mehrerer kritischer Defekte umfasst.

6. Verfahren nach Anspruch 1, wobei das Erkennen (710) der Notwendigkeit Empfangen eines Befehls von einem Benutzer über eine Benutzerschnittstelle in der Bakenvorrichtung (200) umfasst.

7. Verfahren nach Anspruch 1, wobei das Erkennen (710) der Notwendigkeit Erkennen eines Ablaufs eines Zeitgebers umfasst.

8. Verfahren nach einem der Ansprüche 1 bis 7, wobei der Server (300) einen zweiten Funkfrequenzsendeempfänger umfasst, und wobei die Verwendung (750) des Funkfrequenzsendeempfängers Senden der Informationen an den zweiten Funkfrequenzsendeempfänger umfasst.

9. Verfahren nach einem der Ansprüche 1 bis 7, wobei die Verwendung (550) des Funkfrequenzsendeempfängers Weiterleiten der Informationen an eine ähnliche Bake (201) umfasst, die ungehinderten Zugriff auf das verdrahtete Backbone-Netzwerk (250) hat.

10. Verfahren nach einem der Ansprüche 1 bis 7, wobei die Verwendung (750) des Funkfrequenzsendeempfängers Weiterleiten der Informationen über eine Serie ähnlicher Baken (201, 202) zu einer ähnlichen Bake umfasst, die ungehinderten Zugriff auf das verdrahtete Backbone-Netzwerk (250) hat.

11. Verfahren nach einem der vorstehenden Ansprüche, ferner Aktivieren eines visuellen Signals umfassend, wenn festgestellt wird, dass ein oder mehrere der kritischen Defekte vorliegen.

12. Verfahren nach einem der vorstehenden Ansprüche, ferner Verwenden des Funkfrequenzsendeempfängers zum Empfangen zusätzlicher Informationen von dem Server (300) umfassend, wobei zusätzliche Informationen von einer dritten Bakenvorrichtung (202, 203) weitergeleitet wurden.

13. Computerprogrammprodukt, Codemittel umfassend, die konfiguriert sind, einen Prozessor zu veranlassen, das Verfahren nach einem der vorstehenden Ansprüche durchzuführen.

14. Bakenvorrichtung (200) zur Verwendung in einem Lokalisierungssystem für Rufe von Pflegepersonal, wobei die Bakenvorrichtung (200) einen ersten Funkfrequenzsendeempfänger umfasst, der ausgelegt ist, Identifikationsmeldungen zu empfangen, die von einem Identifikationstag (100) ausgehen, und eine Netzwerkschnittstelle, die mit einem verdrahteten Backbone-Netzwerk (250) verbindbar ist;
wobei die Bakenvorrichtung (200) konfiguriert ist, eine Notwendigkeit zu erkennen, die Informationen an einen Server weiterzuleiten, **dadurch gekennzeichnet, dass**:
die Bakenvorrichtung (200) ferner einen Diagnostikagenten umfasst, der konfiguriert ist, bestimmte kritische Defekte des verdrahteten Backbone-Netzwerks zu erkennen, wobei die Bakenvorrichtung konfiguriert ist, die Informationen über das verdrahtete Backbone-Netzwerk zu senden, wenn festgestellt wird, dass keiner der kritischen Defekte vorliegt;
die Bakenvorrichtung (200) ferner konfiguriert ist, den ersten Funkfrequenzsendeempfänger zu verwenden, um die Informationen an den Server (300) zu senden, wenn der Diagnostikagent das Vorliegen von einem der kritischen Defekte des verdrahteten Backbone-Netzwerks (250) erkennt; und
die Bakenvorrichtung (200) ferner konfiguriert ist, in regelmäßigen Intervallen eine Bakenmeldung über eines von dem ersten Funkfrequenzsendeempfänger, einem Magnetinduktionssendeempfänger und einem Ultraschallsender zu senden; und die Dauer der Intervalle zu erhöhen, wenn der Diagnostikagent das Vorliegen von einem der kritischen Defekte des verdrahteten Backbone-Netzwerks (250) erkennt.

## Revendications

1. Procédé pour distribuer des informations dans un système de localisation d'appel d'infirmière, le procédé comprenant au niveau d'un premier appareil formant balise (200) conçu pour recevoir des messages d'identification provenant d'une étiquette d'identification (100) via un premier émetteur-récepteur radiofréquence et relié à un réseau fédérateur câblé (250) :
- la détection (710) d'un besoin de retransmettre lesdites informations à un serveur (300) ; **caractérisé par** :
- le test (720) dudit réseau fédérateur câblé (250) en ce qui concerne la présence de certains défauts critiques ;
- si (730) aucun desdits défauts critiques n'est trouvé comme étant présent, la transmission (740) desdites informations audit serveur (300) sur ledit réseau fédérateur câblé (250) ; et
- autrement, l'utilisation (750) dudit émetteur-récepteur radiofréquence pour transmettre lesdites informations audit serveur (300) ;
dans lequel le procédé comprend en outre :
- la transmission (810) à intervalles réguliers d'un message de balise en provenance dudit premier appareil formant balise (200) via l'un dudit émetteur-récepteur radiofréquence, d'un émetteur-récepteur à induction magnétique et d'un émetteur à ultrasons ; et, lors de la détection (730) d'un ou plusieurs desdits certains défauts critiques dudit réseau fédérateur câblé (250) au niveau de ladite première balise (200) ou au niveau d'une balise similaire avec une portée de communication sans fil de ladite première balise (200), l'augmentation (820) de la durée desdits intervalles.

2. Procédé selon la revendication 1, dans lequel ladite détection (710) dudit besoin comprend la réception d'un message comprenant des informations à retransmettre audit serveur (300).

3. Procédé selon la revendication 2, dans lequel ledit message est un message d'identification provenant de ladite étiquette d'identification (100).

4. Procédé selon la revendication 2, dans lequel ledit message est un message transmis par un appareil formant balise similaire (201).

5. Procédé selon la revendication 1, dans lequel ladite détection (710) dudit besoin comprend la détection d'une présence d'un ou plusieurs desdits défauts critiques.

6. Procédé selon la revendication 1, dans lequel ladite détection (710) dudit besoin comprend la réception d'un ordre provenant d'un utilisateur via une interface utilisateur comprise dans ledit appareil formant balise (200).

7. Procédé selon la revendication 1, dans lequel ladite détection (710) dudit besoin comprend la détection d'une expiration d'une minuterie.

8. Procédé selon l'une quelconque des revendications 1 à 7, dans lequel ledit serveur (300) comprend un second émetteur-récepteur radiofréquence, et dans lequel ladite utilisation (750) dudit émetteur-récepteur radiofréquence comprend la transmission desdites informations audit second émetteur-récepteur radiofréquence.

9. Procédé selon l'une quelconque des revendications 1 à 7, dans lequel ladite utilisation (550) dudit émetteur-récepteur radiofréquence comprend la retransmission desdites informations à une balise similaire (201) qui a un accès sans entrave audit réseau fédérateur câblé (250).

10. Procédé selon l'une quelconque des revendications 1 à 7, dans lequel ladite utilisation (750) dudit émetteur-récepteur radiofréquence comprend la retransmission desdites informations via une série de balises similaires (201, 202) à une balise similaire qui a un accès sans entrave audit réseau fédérateur câblé (250).

11. Procédé selon l'une quelconque des revendications, comprenant en outre, si un ou plusieurs desdits défauts critiques sont trouvés comme étant présents, l'activation d'un signal visuel.

12. Procédé selon l'une quelconque des revendications précédentes, comprenant en outre l'utilisation dudit émetteur-récepteur radiofréquence pour recevoir des informations supplémentaires en provenance dudit serveur (300), lesdites informations supplémentaires ayant été retransmises par un troisième appareil formant balise (202, 203).

13. Produit formant programme informatique comprenant un moyen formant code configuré pour amener un processeur à effectuer le procédé selon l'une quelconque des revendications précédentes.

14. Appareil formant balise (200) à utiliser dans un système de localisation d'appel d'infirmière, l'appareil formant balise (200) comprenant un premier émetteur-récepteur radiofréquence conçu pour recevoir des messages d'identification provenant d'une étiquette d'identification (100), et une interface réseau pouvant être reliée à un réseau fédérateur câblé (250) ;
l'appareil formant balise (200) étant configuré pour détecter un besoin de retransmettre lesdites informations à un serveur ; **caractérisé en ce que** :
ledit appareil formant balise (200) comprend en outre un agent de diagnostic configuré pour détecter certains défauts critiques dudit réseau fédérateur câblé, ledit appareil formant balise étant configuré pour transmettre lesdites informations sur ledit réseau fédérateur câblé si aucun desdits défauts critiques n'est trouvé comme étant présent ;
ledit appareil formant balise (200) est en outre configuré pour utiliser ledit premier émetteur-récepteur radiofréquence pour transmettre lesdites informations audit serveur (300) si ledit agent de diagnostic détecte la présence de l'un desdits défauts critiques dudit réseau fédérateur câblé (250) ; et
ledit appareil formant balise (200) est en outre configuré pour transmettre à intervalles réguliers un message de balise via l'un dudit premier émetteur-récepteur radiofréquence, d'un émetteur-récepteur à induction magnétique et d'un émetteur à ultrasons ; et pour augmenter la durée desdits intervalles si ledit agent de diagnostic détecte la présence de l'un desdits défauts critiques dudit réseau fédérateur câblé (250).
